# EUROPÄISCHE PATENTSCHRIFT

(11) **EP 0 985 661 B1**
(45) Veröffentlichungstag und Bekanntmachung des Hinweises auf die Patenterteilung: **06.02.2002**
(21) Anmeldenummer: 99117237.0
(22) Anmeldetag: 02.09.1999
(51) Int. Cl.: C07C 279/14, C07C 277/08

(54) **Verfahren zur Herstellung von Kreatin oder Kreatinmonohydrat**
Process for the preparation of creatine or creatine monohydrate
Procédé de préparation de créatine ou monohydrate de créatine

(30) Priorität: 10.09.1998 DE 19841525
(43) Veröffentlichungstag der Anmeldung: 15.03.2000
(73) Patentinhaber: BASF AKTIENGESELLSCHAFT, 67056 Ludwigshafen (DE)
(72) Erfinder: Kessel, Knut, Dr., 68161 Mannheim (DE); Scherr, Günter, Dr., 67065 Ludwigshafen (DE); Kluge, Michael, Dr., 67071 Ludwigshafen (DE); Biedermann, Norbert, Dr., 67098 Bad Dürkheim (DE); Greindl, Thomas, Dr., 67098 Bad Dürkheim (DE); Bogenstätter, Thomas, Dr., 67098 Bad Dürkheim (DE); Hähnlein, Wolfgang, Dr., 67251 Freinsheim (DE)

(56) Entgegenhaltungen:
- EP-A- 0 754 679
- US-A- 2 574 510

## Beschreibung

Die vorliegende Erfindung betrifft ein Verfahren zur Herstellung von Kreatin oder Kreatin-monohydrat durch Umsetzung von Natrium- oder Kaliumsarkonisat mit Cyanamid.

Kreatin [N-Amidinosarkosin] ist ein vorwiegend im Muskelgewebe der Wirbeltiere vorkommender Naturstoff. Als Kreatinphosphat bildet er eine wichtige Energiereserve des Muskels. Aus diesem Grund wird Kreatin als Nahrungsergänzungsmittel besonders von Sportlern verwendet.

Die technischen Verfahren zur Herstellung von Kreatin erfolgen durch Umsetzung von Sarkosin bzw. Natrium- oder Kaliumsarkosinat mit Cyanamid oder O-Methylisoharnstoff und sind u.a. beschrieben in Ullmann's Encyclopedia of Industrial Chemistry, 5. Auflage, Band A 12, 552, VCH-Verlagsgesellschaft, Weinheim (1987), sowie in den folgenden Patentschriften US 2,654,779, EP-A-0 754 679 und JA 53-077364 und der dort zitierten weiterführenden Literatur.

Gemäß dem o.g. Stand der Technik erfolgt die Umsetzung unter basischen Bedingungen, vorteilhafterweise bei einem pH-Wert zwischen 9 und 10. Bei der Umsetzung von wäßriger, technischer Natrium- oder Kaliumsarkosinatlösung, die einen pH-Wert größer 10 hat, erfolgt die Einstellung des pH-Wertes durch Zugabe von anorganischen oder organischen Säuren, insbesondere durch Zugabe wäßriger Salz- oder Schwefelsäure sowie durch Essigsäure oder Ameisensäure.

Die stark korrosiven Eigenschaften von Salzsäure machen den Einsatz teurer Werkstoffe, beispielsweise Emailreaktoren oder spezielle Glasapparaturen, erforderlich. Die pH-Wert Regelung mittels Schwefelsäure kann zur Ausfällung von Natriumsulfat und somit zu einer Beeinträchtigung der Produktreinheit von Kreatin führen. Die Verwendung organischer Säuren wie Ameisensäure und Essigsäure führt zu einer erhöhten Abwasserbelastung durch organischen Kohlenstoff, dessen Gehalt im Abwasser großtechnischer Prozesse aus ökologischen Gründen möglichst gering sein soll. Außerdem erfordert die pH-Wert Einstellung mit den o.g. Säuren einen erheblichen Aufwand an Meß- und Regeltechnik.

Es war daher Aufgabe, ein kostengünstiges und einfach durchführbares Verfahren zur Herstellung von Kreatin oder Kreatin-monohydrat bereitzustellen, das die oben geschilderten Nachteile nicht aufweist.

Diese Aufgabe wurde gelöst durch ein Verfahren zur Herstellung von Kreatin oder Kreatin-monohydrat durch Umsetzung von Natrium- oder Kaliumsarkosinat mit Cyanamid bei einer Temperatur von 20 bis 150°C und einem pH-Wert von 7,0 bis 14,0, dadurch gekennzeichnet, daß die pH-Wert Einstellung mit Kohlensäure vorgenommen wird.

Überraschenderweise hat sich gezeigt, daß zur Einstellung des pH-Wertes von 7,0 bis 14,0 eine einmalige Zugabe von Kohlensäure zur wäßrigen Natrium- oder Kaliumsarkosinatlösung ausreicht. Durch die Pufferwirkung des Systems Sarkosinat/H₂CO₃ ist auch während der anschließenden Umsetzung mit Cyanamid keine weitere Zugabe von Kohlensäure zur pH-Wert Regulierung nötig. Somit kann auf eine aufwendige, pH-Wert-geregelte Dosiervorrichtung verzichtet werden, wodurch das Gesamtverfahren zur Herstellung von Kreatin wesentlich vereinfacht wird.

Die zur pH-Wert Einstellung verwendete Kohlensäure kann durch Einleiten von gasförmigem Kohlendioxid oder durch Zugabe von festem Kohlendioxid, dem sogenannten Trockeneis, hergestellt werden. Bevorzugt zur Herstellung der erindungsgmäß verwendeten Kohlensäure ist die Verwendung von gasförmigem Kohlendioxid.

Der pH-Wert bei der Umsetzung von Natrium- oder Kaliumsarkosinat mit Cyanamid liegt im alkalischen Bereich zwischen 7,0 und 14,0, bevorzugt zwischen 8,0 und 12,0, besonders bevorzugt zwischen 9,0 und 10,0.

Die Reaktionstemperatur liegt im Bereich von 20 bis 150°C, bevorzugt von 30 bis 120°C, besonders bevorzugt im Bereich von 50 bis 100°C, wobei gegebenenfalls unter Druck gearbeitet wird.

Bei der wäßrigen Natrium- oder Kaliumsarkosinatlösung handelt es sich um eine 5 bis 60 gew.-%ige, bevorzugt 35 bis 45 gew.-%ige, wäßrige Lösung.

Cyanamid wird vorzugsweise in Form einer 50 gew.-%igen wäßrigen Lösung eingesetzt.

Das Molverhältnis Cyanamid zu Natrium- oder Kaliumsarkosinat kann in weiten Grenzen variiert werden. Vorzugsweise liegt dieses Verhältnis im Bereich zwischen 1:3 und 3:1, besonders bevorzugt zwischen 1:1 und 1:1,5.

Die erfindungsgemäße Umsetzung von Natrium- oder Kaliumsarkosinat mit Cyanamid kann sowohl diskontinuierlich als auch kontinuierlich erfolgen.

In einer bevorzugten Ausführungsform wird eine wäßrige, technische Natrium- oder Kaliumsarkosinatlösung mit Kohlensäure auf einen pH-Wert zwischen 7,0 und 14,0. bevorzugt zwischen 8,0 und 12,0, besonders bevorzugt zwischen 9,0 und 10,0, eingestellt. Die hierfür verwendete Kohlensäure wird durch Einleiten von CO₂ in die wäßrige Sarkosinatlösung bei Temperaturen zwischen 10 und 50°C, bevorzugt zwischen 20 und 40°C, hergestellt. Anschließend wird die auf den gewünschten pH-Wert eingestellte Lösung mit Cyanamid, insbesondere mit einer 40 bis 60 gew.-%igen wäßrigen Cyanamidlösung, über einen Zeitraum von 0,5 bis 8 Stunden, bevorzugt 1 bis 5 Stunden, versetzt. Die Temperatur während der Zugabe liegt im Bereich von 20 bis 150°C, bevorzugt von 30 bis 120°C, besonders bevorzugt im Bereich von 50 bis 100°C. Auf eine weitere pH-Wert Regulierung während der Cyanamid-Zugabe durch Kohlensäure kann bei dieser Reaktionsdurchführung verzichtet werden. Nach der Zugabe von Cyanamid ist es von Vorteil, das Reaktionsgemisch zur vollständigen Umsetzung des Cyanamids bei der o.g. Temperatur nachzurühren.

Es ist auch möglich, die Umsetzung in einem wäßrig-organischen Lösungsmittelsystem durchzuführen, beispielsweise in einem wäßrig-alkoholischen System in Gegenwart eines Alkohols, wie z.B. Methanol, Ethanol oder Isopropanol.

Die Isolierung von Kreatin oder Kreatin-monohydrat erfolgt in an sich bekannter Weise. So läßt sich beispielsweise durch Abkühlen der Reaktionslösung auf -20 bis 60°C, insbesondere 0 bis 40°C, das Wertprodukt kristallin erhalten. Nach Filtration kann gegebenenfalls durch eine weitere Umkristallisation die Reinheit verbessert werden. Es ist aber auch möglich, das Produkt mittels Extraktion aus dem Reaktionsgemisch zu entfernen, um es anschließend durch Destillation oder Kristallisation sauber zu isolieren.

In dem folgenden Beispiel wird das Verfahren zur Herstellung von Kreatin näher erläutert.

### Beispiel: Herstellung von Kreatin

In einem 2-l-Dreihalskolben mit mechanischem Rührer, Heizbad, Rückflußkühler und Gaseinleitungsrohr wurden 1315 g einer 40 %igen wäßrigen Natriumsarkosinatlösung (pH 14) vorgelegt und durch Einleiten von Kohlendioxidgas auf pH 9 eingestellt. Nach Erwärmen der Lösung auf 75°C wurden innerhalb von 4 Stunden 338 g einer 50 %igen wäßrigen Cyanamidlösung zugegeben. Die Temperatur wurde während der gesamten Zugabezeit konstant bei 75°C gehalten. Der pH-Wert blieb ohne weitere CO₂-Eingasung zwischen 9 und 10. Nach der Cyanamidzugabe wurde die Reaktionslösung 2 Stunden nachgerührt und anschließend auf 35°C abgekühlt. Das ausgefallene Kreatin wurde abfiltriert, mit Wasser gewaschen und getrocknet. Man erhielt 480 g Kreatin-monohydrat mit einer Kristall-Ausbeute von 75 %.

## Patentansprüche

1. Verfahren zur Herstellung von Kreatin oder Kreatin-monohydrat durch Umsetzung von Natrium- oder Kaliumsarkosinat mit Cyanamid bei einer Temperatur von 20 bis 150°C und einem pH-Wert von 7,0 bis 14,0, **dadurch gekennzeichnet, daß** die pH-Wert Einstellung mit Kohlensäure vorgenommen wird.

2. Verfahren nach Anspruch 1, **dadurch gekennzeichnet, daß** die für die pH-Wert Einstellung verwendete Kohlensäure durch Einleiten von CO₂-Gas oder durch Zugabe von Trockeneis hergestellt wird.

3. Verfahren nach einem der Ansprüche 1 und 2, **dadurch gekennzeichnet, daß** man den pH-Wert auf 9,0 bis 10,0 einstellt.

4. Verfahren nach einem der Ansprüche 1 bis 3, **dadurch gekennzeichnet, daß** die Umsetzung bei einer Temperatur von 50 bis 100°C erfolgt.

5. Verfahren nach einem der Ansprüche 1 bis 4, **dadurch gekennzeichnet, daß** das Natrium- oder Kaliumsarkosinat in Form einer 35 bis 45 gew.-%igen wäßrigen Lösung eingesetzt wird.

6. Verfahren nach einem der Ansprüche 1 bis 5, **dadurch gekennzeichnet, daß** man Cyanamid in Form einer 40 bis 60 gew.-%igen, wäßrigen Lösung einsetzt.

7. Verfahren nach Anspruch 1, **dadurch gekennzeichnet, daß** man
a) eine wäßrige, technische Natrium- oder Kaliumsarkosinatlösung durch Einleiten von CO₂ auf einen pH-Wert von 7,0 bis 14,0 einstellt und
b) diese Lösung bei einer Temperatur von 20 bis 150°C mit einer 40 bis 60 Gew.-%igen wäßrigen Lösung von Cyanamid versetzt, wobei während der Cyanamid-Zugabe auf eine weitere pH-Wert Regulierung durch Kohlensäure verzichtet werden kann.

## Claims

1. A process for the preparation of creatine or creatine monohydrate by reaction of sodium or potassium sarcosinate with cyanamide at a temperature from 20 to 150°C and a pH from 7.0 to 14.0, which comprises carrying out the pH adjustment with carbonic acid.

2. A process as claimed in claim 1, wherein the carbonic acid used for the pH adjustment is prepared by introduction of CO₂ gas or by addition of dry ice.

3. A process as claimed in one of claims 1 and 2, wherein the pH is adjusted to 9.0 to 10.0.

4. A process as claimed in one of claims 1 to 3, wherein the reaction is carried out at a temperature from 50 to 100°C.

5. A process as claimed in one of claims 1 to 4, wherein the sodium or potassium sarcosinate is employed in the form of a 35 to 45% strength by weight aqueous solution.

6. A process as claimed in one of claims 1 to 5, wherein cyanamide is employed in the form of a 40 to 60% strength by weight aqueous solution.

7. A process as claimed in claim 1, which comprises
a) adjusting an aqueous, industrial sodium or potassium sarcosinate solution to a pH from 7.0 to 14.0 by introduction of CO₂ and
b) treating this solution with a 40 to 60% strength by weight aqueous solution of cyanamide at a temperature from 20 to 150°C, it being possible to dispense with a further pH regulation by carbonic acid during the cyanamide addition.

## Revendications

1. Procédé pour la préparation de la créatine ou de son monohydrate par réaction du sarcosinate de sodium ou de potassium avec le cyanamide à une température de 20 à 150°C et un pH de 7,0 à 14,0, **caractérisé par le fait que** l'on règle le pH à l'aide de l'acide carbonique.

2. Procédé selon la revendication 1, **caractérisé par le fait que** l'acide carbonique servant au réglage du pH est obtenu par injection de CO₂ gazeux ou par addition de glace carbonique.

3. Procédé selon l'une des revendications 1 et 2, **caractérisé par le fait que** l'on règle le pH à un niveau de 9,0 à 10,0.

4. Procédé selon l'une des revendications 1 à 3, **caractérisé par le fait que** la réaction est réalisée à une température de 50 à 100°C.

5. Procédé selon l'une des revendications 1 à 4, **caractérisé par le fait que** le sarcosinate de sodium ou de potassium est mis en oeuvre à l'état de solution aqueuse à une concentration de 35 à 45 % en poids.

6. Procédé selon l'une des revendications 1 à 5, **caractérisé par le fait que** le cyanamide est mis en oeuvre à l'état de solution aqueuse à une concentration de 40 à 60 % en poids.

7. Procédé selon la revendication 1, **caractérisé par le fait que**
a) on règle à un pH de 7,0 à 14,0 par injection de CO₂ une solution aqueuse de sarcosinate de sodium ou de potassium technique et
b) on ajoute à cette solution, à une température de 20 à 150°C, une solution aqueuse de cyanamide à une concentration de 40 à 60 % en poids, et l'on peut alors éviter au cours de l'addition du cyanamide un autre réglage de pH à l'aide de l'acide carbonique.
